# EUROPEAN PATENT SPECIFICATION

(11) **EP 3 603 691 B1**
(45) Date of publication and mention of the grant of the patent: **08.09.2021**
(21) Application number: 18775089.8
(22) Date of filing: 23.03.2018
(51) Int. Cl.: A61M 1/16

(54) **DIALYSATE FOR HEMODIALYSIS**
DIALYSAT ZUR HÄMODIALYSE
DIALYSAT POUR HÉMODIALYSE

(30) Priority: 30.03.2017 JP 2017068858
(43) Date of publication of application: 05.02.2020
(73) Proprietor: Nihon Trim Co., Ltd., Osaka 531-0076 (JP); Tohoku University, Sendai-shi, Miyagi 980-8577 (JP)
(72) Inventor: NAKAYAMA Masaaki, Sendai-shi Miyagi 980-8577 (JP); KABAYAMA Shigeru, Osaka-shi Osaka 531-0076 (JP)
(74) Representative: Agasse, Stéphane
(86) International application number: PCT/JP2018/011938
(87) International publication number: WO 2018/181074

(56) References cited:
- EP-A1- 1 982 736
- WO-A1-2010/103894
- JP-A- 2010 063 629
- JP-A- 2014 161 605
- JP-A- 2015 003 871

## Description

### TECHNICAL FIELD

The present invention relates to dialysate for hemodialysis.

### BACKGROUND ART

Hemodialysis is known as an effective therapy for patients suffering from kidney failure with a reduced renal function and thus having difficulties in adjusting the amount of water in the body and in urination for removing toxic substances including wastes, such as urea, from the body.

The hemodialysis is a therapy repeating the following procedure: blood is sucked out of the body of a patient using a blood pump and brought into contact with dialysate through a dialyzer to remove toxic substances and moisture from the blood by utilizing a diffusion phenomenon caused by a concentration gradient; and the blood is then returned to the body.

As the dialysate, for example, dialysate containing dissolved hydrogen is produced by the following method (see, e.g., Patent Document 1). The water (hereinafter referred to as "reverse osmosis water") filtered and purified by a reverse osmosis (RO) membrane is bubbled, and thereby brought into contact with, hydrogen gas. The hydrogen is therefore dissolved into the reverse osmosis water.

EP1982736A1 discloses a treated water for preparing a dialysis solution, said water being treated by dissolving hydrogen at a concentration of 50 to 600 ppb and by having a specific pH. This water is used to prepare a dialysis solution by diluting therein a dialysis base agent comprising, among others, glucose, said dialysis solution inevitably including glucose degradation products generated by decomposition and autooxidation of the glucose contained in the dialysis solution.

JP2015003871A describes a dialysate composition that may comprise dissolved hydrogen in a content of more than 100 ppb.

### CITATION LIST

### PATENT DOCUMENT

PATENT DOCUMENT 1: Japanese Unexamined Patent Publication No. 2016-13349

### SUMMARY OF THE INVENTION

### TECHNICAL PROBLEM

In recent years, it is known that dialysis patients undergo oxidative stress during hemodialysis using the typical dialysate described above. This is believed to be caused by active oxygen generated during the dialysis. The oxidative stress causes blood pressure fluctuations during or right after the treatment or a complication such as itching or fatigue after the treatment. It is thus necessary to eliminate the cause, which is the active oxygen, to reduce the oxidative stress.

However, it is difficult to eliminate the active oxygen by using the dialysate described in Patent Document 1, which makes it impossible to reduce the oxidative stress.

It is therefore an objective of the present invention to provide dialysate capable of eliminating active oxygen and reducing oxidative stress.

### SOLUTION TO THE PROBLEM

In order to achieve the objective, dialysate for hemodialysis according to the present invention contains hydrogen dissolved at a concentration ranging from 30 ppb to 80 ppb.

### ADVANTAGES OF THE INVENTION

The present invention improves an antioxidant effect on dialysis patients and thus reduces various complications caused by oxidative stress.

### BRIEF DESCRIPTION OF THE DRAWINGS

FIG. 1 is a conceptual diagram illustrating a configuration of a system for generating dialysate for hemodialysis according to an embodiment of the present invention.
FIG. 2 illustrates an electrolyzer of an electrolyzed water generator in the system for generating dialysate for hemodialysis according to the embodiment of the present invention.
FIG. 3 illustrates a dialysis machine according to the embodiment of the present invention.
FIG. 4 illustrates the percentage of patients complaining of intensity of itching in an experimental group.
FIG. 5 illustrates the percentage of patients complaining of frequency of itchiness in the experimental group.
FIG. 6 illustrates the percentage of patients complaining of intensity of itching in a comparative group.
FIG. 7 illustrates the percentage of patients complaining of frequency of itchiness in the comparative group.
FIG. 8 illustrates the percentage of patients complaining of intensity of fatigue in the experimental group.
FIG. 9 illustrates the percentage of patients complaining of intensity of fatigue in the comparative group.
FIG. 10 illustrates changes in defined daily dosages (DDDs) of antihypertensives (depressor) in the experimental and comparative groups.

### DESCRIPTION

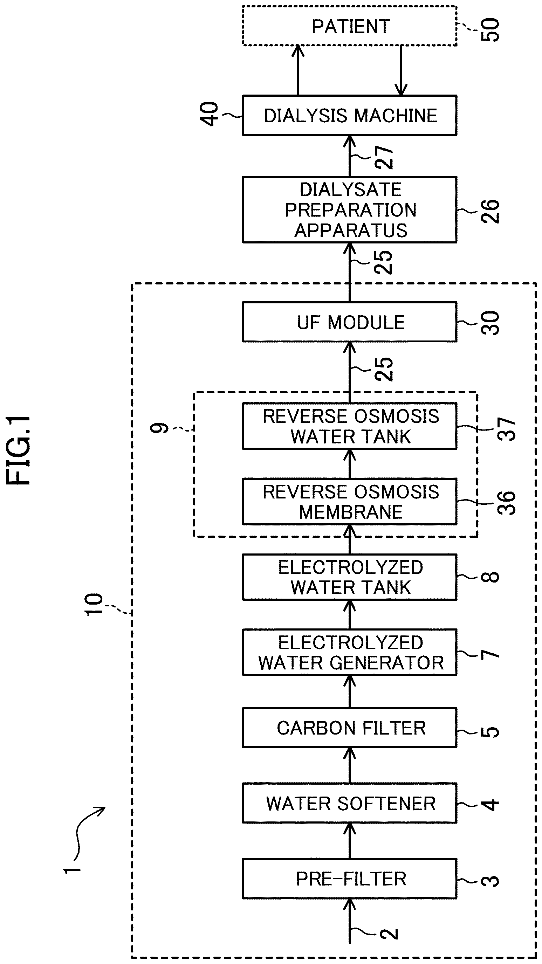

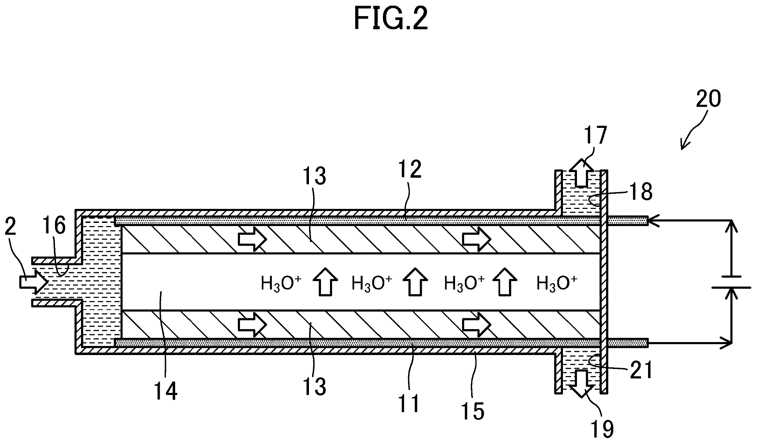

FIG. 1 is a conceptual diagram illustrating a configuration of a system for generating dialysate for hemodialysis according to an embodiment of the present invention. FIG. 2 illustrates an electrolyzer of an electrolyzed water generator in the system for generating dialysate for hemodialysis according to the embodiment of the present invention.

The "dialysate for hemodialysis" in the present invention may also be used (although this is not part of the present invention) as transfusion such as a replacement liquid or a substitution fluid in other blood purification therapies such as hemofiltration and hemodiafiltration.

Components suitable for the dialysate for hemodialysis may be various electrolytes such as sodium ions (Na⁺), potassium ions (K⁺), calcium ions (Ca²⁺), magnesium ions (Mg²⁺), chloride ions (Cl⁻), acetate ions (CH₃COO⁻), and bicarbonate ions (HCO₃⁻), as well as glucose, lactic acids, citric acids, saccharides, polysaccharides, and amino acids.

The "dialysate for hemodialysis" in the present invention will be hereinafter simply referred to as "dialysate."

As shown in FIG. 1, a dialysate production system 1 which is not part of the present invention includes a generator 10 of water for preparing dialysate and a dialysate preparation apparatus 26.

The generator 10 of water for preparing dialysate includes a pre-filter 3, a water softener 4 connected to the pre-filter 3, a carbon filter (activated carbon treatment apparatus) 5 connected to the softener 4, an electrolyzed water generator 7 connected to the carbon filter 5, an electrolyzed water tank 8 connected to the electrolyzed water generator 7, a reverse osmosis membrane treatment system 9 connected to the electrolyzed water tank 8, and an ultra-filter (UF) module 30 connected to the reverse osmosis membrane treatment system 9.

The pre-filter 3 is for removing impurities (e.g., iron rust or sand particles) from raw water 2 (i.e., hard water containing dissolved solids such as calcium ions and magnesium ions that are hardness components).

The water softener 4 is for removing the hardness components from the raw water 2 by substitution reaction through ion exchange to obtain soft water. In this embodiment, the raw water 2 may be, for example, tap water, well water, or ground water.

The carbon filter 5 is for removing, for example, residual chlorine, chloramine, or organic substances from the raw water treated by the water softener 4, by physical adsorption using activated carbon as a porous adsorbate.

Each of the water softener 4 and the carbon filter 5 may be of a known type.

The electrolyzed water generator 7 functions as a hydrogen dissolver for performing electrolysis of the raw water 2 processed by the carbon filter 5, thereby generating water (i.e., hydrogen-dissolved water) that contains dissolved hydrogen and is used as water for preparing dialysate.

The electrolyzed water generator 7 according to this embodiment which is not part of the present invention includes an electrolyzer 20 having a solid polymer membrane (solid polymer electrolyte membrane) 14 shown in FIG. 2, which is also not part of the present invention.

As shown in FIG. 2, the electrolyzer 20 not part of the invention includes the solid polymer membrane 14 described above, an anode 11, a cathode 12, and dielectric layers 13. The anode 11 and the cathode 12 are power feeders that face each other with the solid polymer membrane 14 interposed therebetween and supply electricity to the electrolyzer 20. One of the dielectric layers 13 is interposed between the solid polymer membrane 14 and the anode 11, whereas the other is interposed between the solid polymer membrane 14 and the cathode 12.

As shown in FIG. 2, the anode 11 and the cathode 12 are electrically connected to each other. The solid polymer membrane 14, the anode 11, the cathode 12, and the dielectric layers 13 are housed inside an electrolyzer body 15.

As shown in FIG. 2, the electrolyzer body 15 has an inlet passage 16 for introducing, into the electrolyzer body 15, the raw water 2 to be electrolyzed.

The anode 11 and the cathode 12 may be made of titanium or platinum, for example.

The dielectric layers 13 may also be made of titanium or platinum, for example.

The solid polymer membrane 14 has the function of moving the oxonium ions (H₃O⁺) generated by electrolysis at the anode 11 toward the cathode 12.

This solid polymer membrane 14 may be advantageously made of, for example, a fluorine-based resin material containing a sulfonic acid group. More specifically, commercially available products such as Nafion manufactured by DuPont, Flemion manufactured by AGC Chemicals Company, and Aciplex manufactured by AGC Chemicals Company may be advantageously used as the solid polymer membrane 14.

For the electrolysis by the electrolyzed water generator 7 having such a solid polymer membrane 14, the oxonium ions (H₃O⁺) are used as a raw material for generating hydrogen at the cathode 12. No OH⁻ ions are thus generated in the electrolysis process using the electrolyzed water generator 7. Accordingly, there is no change in the pH of the processed water, even if the electrolysis process is performed at a high current value in order to increase the amount of dissolved hydrogen.

This configuration prevents a reduction in concentration of the hydrogen dissolved in the processed water due to the upper limit of the pH. The electrolysis process can thus be performed at a desired high current value, making it possible to increase the concentration of the hydrogen dissolved in the processed water. As a result, processed water containing dissolved hydrogen at a required concentration can be obtained.

The processed water (i.e., hydrogen-dissolved water) 17 generated by the electrolysis process described above is then sent through a water supply channel 18 at the cathode of the electrolyzer body 15 to the electrolyzed water tank 8 connected to the electrolyzed water generator 7. The oxygen-dissolved water 19 generated at the anode by the electrolysis process is discharged outside through a drain channel 21 at the anode of the electrolyzer body 15.

The electrolyzed water tank 8 is for storing the hydrogen-dissolved water generated by the electrolyzed water generator 7.

The reverse osmosis membrane treatment system 9 is for performing the following reverse osmosis membrane treatment. If there are solutions with different concentrations with a semipermeable membrane interposed therebetween, water moves from the solution with a lower concentration to the solution with a higher concentration. With respect to the phenomenon (i.e., osmosis), the reverse osmosis membrane treatment system 9 applies pressure to the solution with the higher concentration to transfer water from the solution with the higher concentration to the solution with the lower concentration, thereby obtaining water that has penetrated into the solution with the lower concentration (i.e., reverse osmosis membrane treatment).

The reverse osmosis membrane treatment system 9 can further remove impurities such as trace metals from the raw water obtained in the series of treatments described above, and thus obtain water (i.e., reverse osmosis water) satisfying the water quality standards defined by ISO 13959 (i.e., standards for water for dialysis).

As shown in FIG. 1, the reverse osmosis membrane treatment system 9 includes a reverse osmosis membrane 36 and a reverse osmosis water tank 37. The reverse osmosis membrane 36 performs the above-described reverse osmosis membrane treatment of the hydrogen-dissolved water generated by the electrolyzed water generator 7. The reverse osmosis water tank 37 stores the reverse osmosis water after the reverse osmosis membrane treatment.

The UF module 30 is for removing bacteria and microorganisms contained in reverse osmosis water 25.

As shown in FIG. 1, the UF module 30 is connected to a dialysate preparation apparatus 26. The reverse osmosis water 25 processed by the UF module 30 is, as the water for preparing dialysate, supplied to the dialysate preparation apparatus 26.

The dialysate preparation apparatus 26 prepares dialysate 27 that is a mixture of the supplied reverse osmosis water 25 and undiluted dialysis. The dialysate 27 is supplied to a dialysis machine 40 connected to the dialysate preparation apparatus 26 to purify the blood of a patient 50. That is, the dialysate preparation apparatus 26 also functions as a dialysate supply apparatus for supplying the prepared dialysate 27 to the dialysis machine 40.

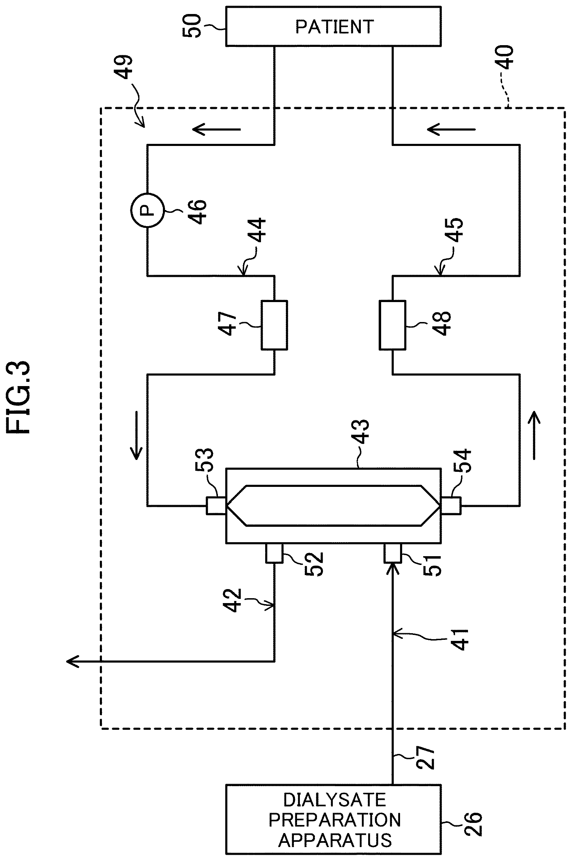

FIG. 3 illustrates a dialysis machine which is not part of the present invention. As shown in FIG. 3, the dialysis machine 40 includes a blood circuit 49 and a dialyzer 43. The blood circuit 49 includes an arterial blood circuit 44 and a venous blood circuit 45. The dialyzer 43 is a blood purifier that is connected to the arterial blood circuit 44 and the venous blood circuit 45 and purifies the blood flowing through the blood circuit 49.

The dialysis machine 40 further includes a dialysate inlet passage 41 and a dialysate outlet passage 42. The dialysate inlet passage 41 is connected to the dialysate preparation apparatus 26 and the dialyzer 43 to introduce the dialysate 27 prepared by the dialysate preparation apparatus 26 into the dialyzer 43. The dialysate outlet passage 42 is connected to the dialyzer 43 to discharge the dialysate 27 introduced into the dialyzer 43, together with wastes in the blood.

The dialysis machine 40 further includes a blood circulation pump 46 and a bubble remover 47 which are provided in the arterial blood circuit 44, and a bubble remover 48 provided in the venous blood circuit 45.

The dialyzer 43 is connected to the dialysate inlet passage 41, the dialysate outlet passage 42, the arterial blood circuit 44, and the venous blood circuit 45 through connectors (couplers) 51 to 54, respectively, shown in FIG. 3. The dialyzer 43 is detachably attached to these connectors 51 to 54.

The pump 46 is driven with arterial and venous puncture needles (both not shown) punctured into the patient 50. Bubbles are then removed from the blood of the patient 50 by the bubble remover 47. The blood is sent to the dialyzer 43 through the arterial blood circuit 44 to be purified by the dialyzer 43. Unless being removed by the bubble remover 47, bubbles may enter the blood and cause embolism.

The dialysis machine 40 also includes a bubble detector (not shown) that raises an alarm and stops the operation (e.g., stops the pump 46 and blocks the blood circuit from the outside to prevent air from entering the inside of the body) when a bubble passes through the detector. In this case, the dialysis machine 40 needs to be checked and restarted, which leads to unstable dialysis and requires a longer time for the dialysis.

Bubbles are then removed by the bubble remover 48 from the blood purified by the dialyzer 43. The blood returns to the body of the patient 50 through the venous blood circuit 45.

A plurality of hollow fibers with a predetermined inner diameter (e.g., 200 µm) are provided inside the dialyzer 43. The inside of the hollow fibers serves as channels for the blood, whereas the gaps between the outer peripheral surface of the hollow fibers and the inner peripheral surface of the housing of the dialyzer 43 serve as channels for the dialysate 27.

The hollow fibers have a large number of pores penetrating their inner and outer peripheral surfaces. The walls of the hollow fibers serve as semipermeable membranes, through which impurities in the blood permeate the dialysate 27.

The present inventors found that the use of the dialysate 27 containing hydrogen dissolved at a concentration ranging from 30 ppb to 80 ppb removes active oxygen generated in dialysis and reduces oxidative stress.

More specifically, the dialysate 27 containing the reverse osmosis water 25 according to the present invention is administered to the patient 50. The dialysate 27 reduces myeloperoxidase in the blood and monocyte chemoattractant protein-1 (MCP-1) and thus reduces the oxidative stress and the inflammatory reaction of the living body. Accordingly, complications such as itching, fatigue, and high-blood pressure caused by the oxidative stress decrease.

In short, the dialysate 27 according to the present invention contains the hydrogen dissolved at the concentration ranging from 30 ppb to 80 ppb to improve the antioxidant effect on the dialysis patient 50, and thus reduces various complications caused by the oxidative stress.

The concentration of the hydrogen dissolved in the dialysate 27 ranges from 30 ppb to 80 ppb. This is because the hydrogen dissolved at a concentration lower than 30 ppb, which is too low, may hinder sufficient reduction in the fatigue or the other problems described above. On the other hand, a concentration of 550 ppb or lower effectively reduces generation of bubbles in the dialysate 27. In particular, a concentration of 230 ppb or lower effectively reduces generation of bubbles in the dialysate 27 and provides safe and stable dialysis.

In Japan, there are more than 300,000 chronic hemodialysis patients, whose low quality of life (QOL), comorbidities, and resultant high rates of death and cardiovascular complications are problematic. The causes of these problems are influenced by the individual medical backgrounds of the patients. In addition, it has become clear that the causes are deeply related to biological disorders caused by the dialysis therapy itself, that is, biological incompatibility (e.g., oxidative stress or microinflammations caused by the contact between the cells of the patients and a dialysis machine or dialysate).

The present invention aims to reduce the biological disorder during the dialysis. We have confirmed in basic research that electrolyzed water containing molecular hydrogen gas has antioxidant and anti-inflammatory effects, and that dialysate prepared using the hydrogen-dissolved water according to the present invention is less cytotoxic.

The present dialysate was developed in view of the above aspects. The clinical use of this dialysate (said clinical use not being part of the invention) achieves an improvement in the QOL of hemodialysis patients and reduction in complications, which can help the patients return to society and family.

The use of the dialysate according to the present invention (use which, as stated above, is not part of the invention) reduces an increase in the subjective symptoms of the fatigue and itching of the skin caused by the dialysis, the blood pressure fluctuations, the prescribed dosages of antihypertensives, and the risk of death and non-fatal cardiovascular complications. The present dialysate clinically safely produces effects on these problems without causing side effects.

### [Examples]

Examples of how to produce the dialysate of the invention and clinical results which avail that it actually works are described below. However, only the dialysate for hemodialysis falls within the scope of the invention.

### (Experimental Group: Examples of 140 Patients)

### <Generation of Dialysate>

Water for preparing dialysate was generated using the generator shown in FIG. 1.

More specifically, raw water (tap water) was first filtered through the pre-filter, and then processed using the water softener and the carbon filter. The processed water was supplied to the electrolyzed water generator to obtain electrolytic hydrogen water (i.e., hydrogen-dissolved water).

Using an electrolytic hydrogen water generator named "TRIM ION HD-24D" manufactured by Nihon Trim Co., Ltd. as the electrolyzed water generator, constant-current electrolysis was performed under the conditions of a flow rate of 12 L/min and a current of 12 A. As a result, electrolytic hydrogen water (hydrogen-dissolved water) with a pH of 9.69 was obtained. The pH was measured by a pH meter with ϕ 260, manufactured by Beckman Coulter Inc.

Using a reverse osmosis purified water production system named "MH500CX" manufactured by Japan Water Systems Corporation as the reverse osmosis membrane treatment system, the electrolytic hydrogen water generated by the electrolysis process was subjected to reverse osmosis membrane treatment to obtain reverse osmosis water (water for preparing dialysate). The electrolytic hydrogen water was supplied at a rate of 500 mL/min to the reverse osmosis membrane treatment system.

Next, the reverse osmosis water (500 ml) was supplied to a dialysis machine, specifically a single-patient monitoring system named "DBB-22B" manufactured by Nikkiso Co., Ltd. to be mixed with undiluted dialysis covered by insurance at a certain ratio according to rules. As a result, the dialysate according to this embodiment was obtained. Undiluted dialysis for artificial kidney named "Kindaly AF-4P" manufactured by Fuso Pharmaceutical Industries, Ltd. was used.

The concentrations of the electrolytes in the produced dialysate were as follows: Na⁺ was 140 mEq/L, K⁺ was 2.0 mEq/L, Ca²⁺ was 2.75 mEq/L, Mg²⁺ was 1.0 mEq/L, Cl⁻ was 112.25 mEq/L, CH₃COO⁻ was 8 mEq/L, HCO₃⁻ was 27.5 mEq/L, and glucose (C₆H₁₂O₆) was 125 mq/dL. The produced dialysate had a pH ranging from 7.2 to 7.4 and an osmotic pressure ratio ranging from 0.95 to 1.00.

### <Evaluation on Itching>

Hemodialysis was performed for twelve months using the produced dialysate. The itching (the intensity and frequency of itching) of patients was evaluated at the start of the test, six months after the start of the test, and twelve months after the start of the test.

More specifically, interviews with the patients themselves were conducted to evaluate the "intensity of itching" based on the following criteria.
Extremely Itchy: High
Moderately Itchy: Moderate
Slightly Itchy: Low
Not Itchy: None

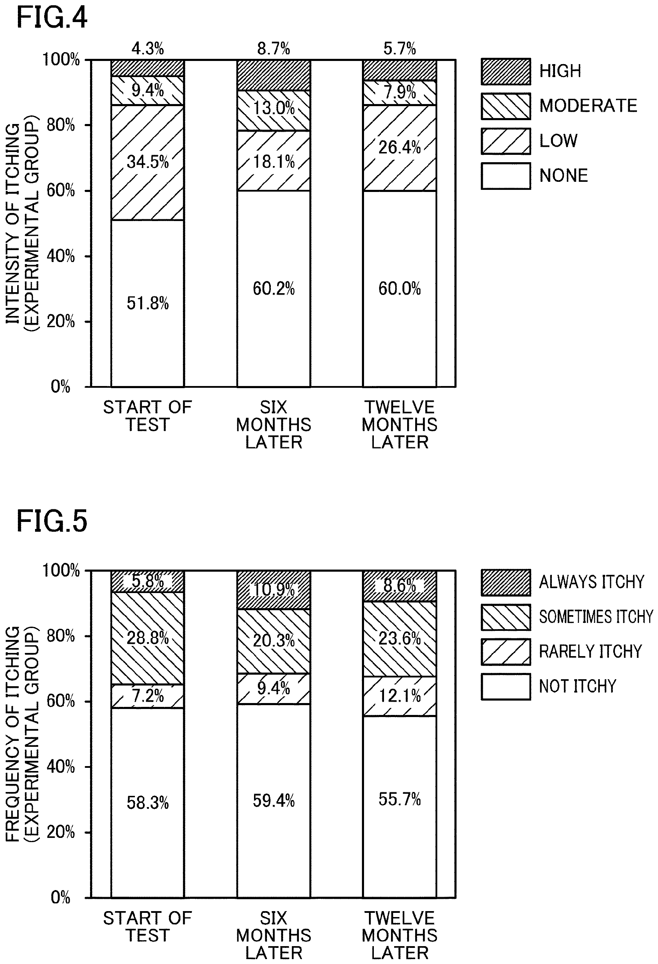

The "frequency of itching" was evaluated based on four criteria: always itchy, sometimes itchy, rarely itchy, and not itchy. The results are shown in Table 1 and FIGS. 4 and 5.

### <Evaluation on Fatigue>

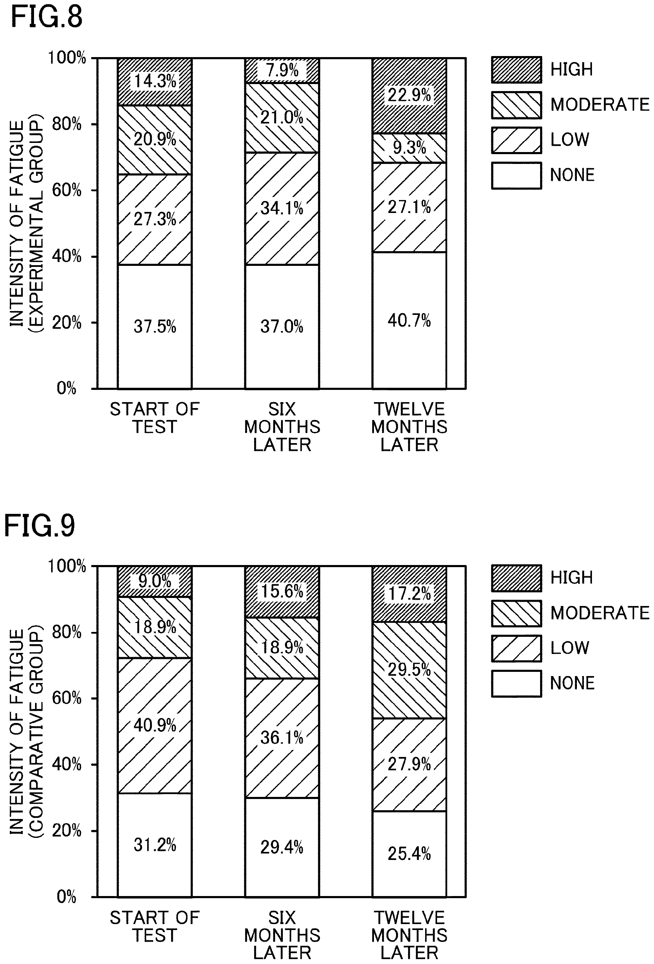

Hemodialysis was performed for twelve months using the produced dialysate. The fatigue (the intensity of fatigue) of patients was evaluated at the start of the test, six months after the start of the test, and twelve months after the start of the test. More specifically, interviews with the patients themselves were conducted to evaluate the "intensity of fatigue" based on the following criteria.
Extremely tired, which inhibits physical activities and requires rest: High
Moderately tired, which limits physical activities: Moderate
Slightly tired, which allows normal physical activities: Low
Not tired, which allows normal physical activities: None
The results are shown in Table 2 and FIG. 8.

### <Evaluation on Dosages of Antihypertensives>

Hemodialysis was performed for twelve months using the produced dialysate. The changes in patients' dosages (daily dosages) of antihypertensives were evaluated at the start of the test, six months after the start of the test, and twelve months after the start of the test.

More specifically, the antihypertensives prescribed to the patients were obtained as defined daily doses (DDDs) which are a converted value for comparison of drugs specified by the World Health Organization. The results are shown in Table 3.

In the evaluations described above, the concentrations of hydrogen dissolved in the reverse osmosis water used and the concentrations of hydrogen in the dialysate were measured using a dissolved hydrogen meter DH-35A manufactured by DKK-TOA CORPORATION. The concentrations of hydrogen dissolved in the dialysate are shown in Tables 1 to 3.

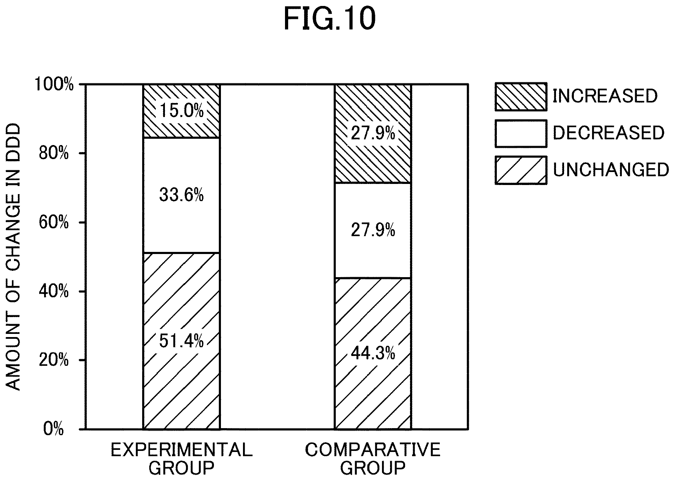

FIG. 10 shows the amount of changes in the defined daily doses (DDDs) between the start of the test and the twelfth month after the start of the test.

### (Comparative Group: Examples of 122 Patients)

The electrolysis process using the electrolytic hydrogen water generator was not performed.

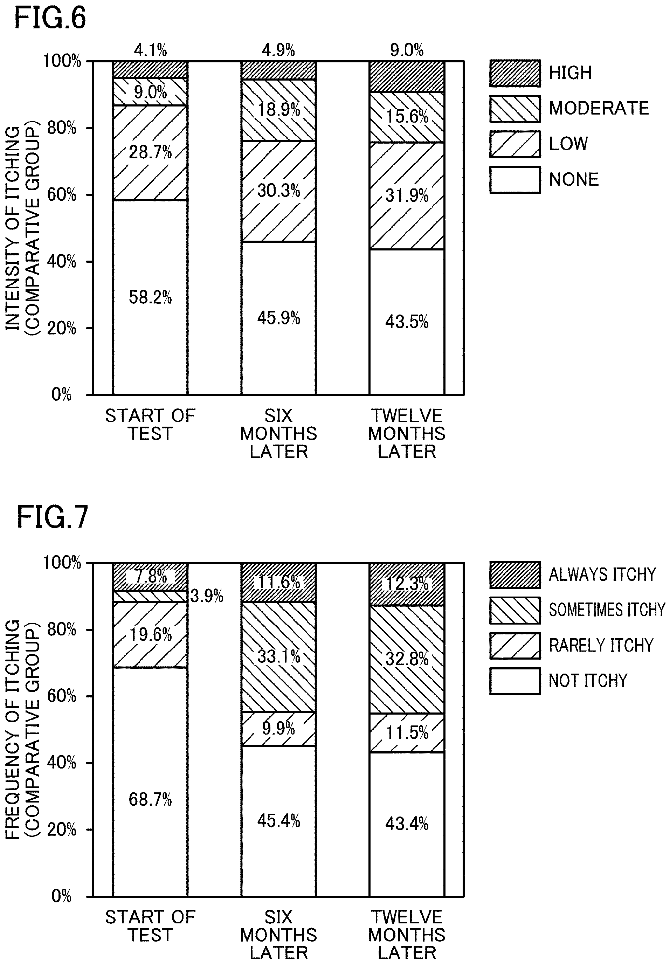

Otherwise, dialysate was prepared in the same manner as in the experimental group. The itching, fatigue, and the prescribed dosages of the antihypertensives were evaluated in the same manner as in the experimental group. The results are shown in Tables 1 to 3 and FIGS. 6, 7, and 9.

**[Table 1]**

| | | Concentration of Dissolved Hydrogen [ppb] | Intensity of Itching (Percentage of Complaining Patients) [%] | | | | Frequency of Itching (Percentage of Complaining Patients) [%] | | | |
|---|---|---|---|---|---|---|---|---|---|---|
| | | Dialysate | High | Moderat e | Low | None | Always Itchy | Sometime s Itchy | Rarely Itchy | Not Itchy |
| Experimenta 1 Group | Start of Test | 30 to 80 | 4.3 | 9.4 | 34.5 | 51.8 | 5.8 | 28.8 | 7.2 | 58.3 |
| | 6 Months Later | 30 to 80 | 8.7 | 13.0 | 18.1 | 60.2 | 10.9 | 20.3 | 9.4 | 59.4 |
| | 12 Months Later | 30 to 80 | 5.7 | 7.9 | 26.4 | 60.0 | 8.6 | 23.6 | 12.1 | 55.7 |
| Comparative Group | Start of Test | 0 | 4.1 | 9.0 | 28.7 | 58.2 | 7.8 | 3.9 | 19.6 | 68.7 |
| | 6 Months Later | 0 | 4.9 | 18.9 | 30.3 | 45.9 | 11.6 | 33.1 | 9.9 | 45.4 |
| | 12 Months Later | 0 | 9.0 | 15.6 | 31.9 | 43.5 | 12.3 | 32.8 | 11.5 | 43.4 |

**[Table 2]**

| | | Concentration of Dissolved Hydrogen [ppb] | Intensity of Fatigue (Percentage of Complaining Patients) [%] | | |
|---|---|---|---|---|---|
| | | Dialysate | High + Moderate | Low | None |
| Experimenta 1 Group | Start of Test | 30 to 80 | 35.2 | 27.3 | 37.5 |
| | 6 Months Later | 30 to 80 | 28.9 | 34.1 | 37.0 |
| | 12 Months Later | 30 to 80 | 32.2 | 27.1 | 40.7 |
| Comparative Group | Start of Test | 0 | 27.9 | 40.9 | 31.2 |
| | 6 Months Later | 0 | 34.5 | 36.1 | 29.4 |
| | 12 Months Later | 0 | 46.7 | 27.9 | 25.4 |

**[Table 3]**

| | | Concentration of Dissolved Hydrogen [ppb] | Dosages of Antihypertensives [Defined Daily Doses] |
|---|---|---|---|
| | | Dialysate | Median (Interquartile Range) |
| Experimental Group | Start of Test | 30 to 80 | 0.64 (0, 1.59) |
| | 6 Months Later | 30 to 80 | 0.57 (0,1.18) |
| | 12 Months Later | 30 to 80 | 0.57 (0,1.18) |
| Comparative Group | Start of Test | 0 | 1.40 (0, 2.55) |
| | 6 Months Later | 0 | 1.33 (0, 2.46) |
| | 12 Months Later | 0 | 1.50 (0, 2.48) |

As shown in Table 1 and FIGS. 4 to 7, the following was found. An increasing percentage of patients using the dialysate in the comparative group complained of more intense and frequent itching with time after the start of the test. In contrast, the percentage of patients who used the dialysate in the experimental group and complained of intense and frequent itching did not increase even twelve months after the start of the test. This means that the dialysate used in the experimental group reduced itching caused by oxidative stress.

As shown in Table 2 and FIGS. 8 and 9, the following was found. An increasing percentage of patients using the dialysate in the comparative group complained of more intense fatigue with time after the start of the test. In contrast, the percentage of patients who used the dialysate in the experimental group and complained of intense fatigue did not increase even twelve months after the start of the test. This means that the dialysate used in the experimental group reduced itching caused by oxidative stress.

As shown in Table 3 and FIG. 10, the following was found. The dosages of antihypertensives in the comparative group did not change, that is, did not decrease even twelve months after the start of the test. In contrast, the dosages of antihypertensives in the experimental group statistically significantly decrease with time. In addition, no particularly problematic side effects were acknowledged in the experimental group.

### (Continuous Examples of Five Patients)

Hemodialysis was continued after twelve months using the prepared dialysate to observe the clinical course during the time. The relationship among the fatigue (the intensity of fatigue) of the patients, the management conditions of blood pressure, and the concentration of hydrogen in the dialysate on the X-th and subsequent months (e.g., 20 months after the start) was evaluated. The results (i.e., the clinical symptoms (of the average of the five patients)) are shown in Table 4.

**[Table 4]**

| Term | X-th Month | X+3 Months | X+6 Months | X+7 Months | X+9 Months | X+13 Months | X+14 Months | X+16 Months |
|---|---|---|---|---|---|---|---|---|
| Concentration of Hydrogen Dissolved in Dialysate [ppb] | 36.3 | 53 | 60 | 35 | 49 | 13 | 13 | 79 |
| Fatigue (Subjective Symptoms after Dialysis) | Low | None | None | Low | None | Some | Some | None |
| Systolic Blood Pressure Fluctuations During Dialysis | Small to Moderate | Small | Small | Small to Moderate | Small | Large | Large | Small |
| Difference between Maximum and Minimum | | | | | | | | |
| Small < 15 mmHg | | | | | | | | |
| Medium < 20 mmHg | | | | | | | | |
| Large ≥ 20 mmHg | | | | | | | | |

As shown in Table 4, the patients using the dialysate containing hydrogen dissolved at a concentration ranging from 30 ppb to 80 ppb had no or slight subjective symptoms of fatigue. In contrast, the patients using the dialysate containing hydrogen dissolved at a concentration lower than 30 ppb had subjective symptoms of fatigue. In addition, the patients using the dialysate containing hydrogen dissolved at a concentration ranging from 30 ppb to 80 ppb had small to moderate blood pressure fluctuations during the dialysis. In contrast, the patients using the dialysate containing hydrogen dissolved at a concentration lower than 30 ppb had large blood pressure fluctuations.

### (Case A of One Patient)

In an example of one patient, the patient had hemodialysis three times in a week using dialysate containing a higher hydrogen concentration. The fatigue (intensity of fatigue) of the patient at each time and the presence or absence of side effects during the dialysis were evaluated. The fatigue was evaluated under the criteria described in the first example. The side effects during the dialysis were evaluated based on the presence or absence of clinically problematic objective and subjective symptoms or signs of such symptoms which were newly triggered during or after the dialysis. More specifically, the presence or absence of abnormal blood pressure fluctuations during the dialysis, lower blood pressure with subjective symptoms, impaired consciousness, skin flare, fever, pain, neurological symptoms, psychiatric symptoms, appearance of unpredictable outliers in biochemical examination of blood, and reduction in the efficiency of the dialysis treatment were evaluated. The results are shown in Table 5.

**[Table 5]**

| Time of Dialyses Using Present Dialysate | | First | Second | Third |
|---|---|---|---|---|
| Concentration of Hydrogen Dissolved in Dialysate [ppb] | Start of Dialysis | 220 | 204 | 186 |
| | End of Dialysis | 227 | 202 | 205 |
| Fatigue (Subjective Symptom After Dialysis) | | None | None | None |
| Side Effects During Dialysis | | None | None | None |

As shown in Table 5, the patient using the dialysate containing hydrogen dissolved at the concentration according to the present disclosure had no particular subjective fatigue symptoms during the dialysis. In particular, no side effects were observed. It is found from the forging that safe and stable dialysis can be performed.

### <Preventive Effect of Total Death and Serious Cardiovascular Diseases>

In an example with 176 patients, hemodialysis was performed using dialysate containing hydrogen dissolved at a concentration ranging from 30 ppb to 80 ppb. In an example with 132 patients, hemodialysis was performed using typical dialysate. In a study, these patient groups have been observed for five years. Out of them, 90 patients in total had an event (e.g., death or a non-fatal, serious cardiovascular disease including a heart disease, a stroke, and amputation for a lower limb ischemic artery disease).

The patient groups were studied based on the Cox proportional hazards model with corrected confounding factors. The hazard ratio to the event was 0.61 (from 0.35 to 0.95 in a 95% confidence interval), that is, statistically significantly low, in the patient group using the dialysate according to the present disclosure, as compared to the patient group using the typical dialysate.

### INDUSTRIAL APPLICABILITY

As described above, the present invention is a dialysate for hemodialysis.

### DESCRIPTION OF REFERENCE CHARACTERS

- 1: Dialysate Production System
- 2: Raw Water
- 3: Pre-Filter
- 4: Water Softener
- 5: Carbon Filter
- 7: Electrolyzed Water Generator (Hydrogen Dissolver)
- 8: Electrolyzed Water Tank
- 9: Reverse Osmosis Membrane Treatment System
- 10: Generator of Water for Preparing Dialysate
- 14: Solid Polymer Membrane
- 11: Anode
- 12: Cathode
- 13: Dielectric Layer
- 15: Electrolyzer Body
- 16: Inlet Passage
- 17: Processed Water
- 18: Water Supply Channel
- 19: Oxygen-Dissolved Water
- 20: Electrolyzer
- 21: Drain Channel
- 26: Dialysate Preparation Apparatus
- 27: Dialysate
- 32: Controller
- 33: Electrolytic Current Determination Means
- 34: Electrolytic Current Supply Means
- 35: Storage Means
- 36: Reverse Osmosis Membrane
- 37: Reverse Osmosis Water Tank
- 40: Dialysis Machine
- 41: Dialysate Inlet Passage
- 43: Dialyzer (Blood Purifier)
- 44: Arterial Blood Circuit
- 45: Venous Blood Circuit
- 50: Patient

## Claims

1. Dialysate for hemodialysis, comprising:
hydrogen dissolved at a concentration ranging from 30 ppb to 80 ppb.

## Patentansprüche

1. Dialysat für Hämodialyse, umfassend:
Wasserstoff, der in einer Konzentration gelöst ist, die von 30 ppb bis 80 ppb reicht.

## Revendications

1. Dialysat pour hémodialyse comprenant de l'hydrogène dissous à une concentration allant de 30 ppb à 80 ppb.
